(19) Europäisches Patentamt
European Patent Office
Office européen des brevets

(11) **EP 3 771 403 A1**

(12) **EUROPEAN PATENT APPLICATION**

(43) Date of publication:
**03.02.2021 Bulletin 2021/05**

(51) Int Cl.:
**A61B 5/024** (2006.01)  **A61B 5/00** (2006.01)

(21) Application number: **20188493.9**

(22) Date of filing: **30.07.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.07.2019 IN 201921031018**

(71) Applicant: **Tata Consultancy Services Limited Maharashtra (IN)**

(72) Inventors:
• **AHMED, NASIMUDDIN**
**700160 Kolkata, West Bengal (IN)**
• **CHOWDHURY, ARIJIT**
**700160 Kolkata, West Bengal (IN)**
• **MUKHOPADHYAY, Shalini**
**700160 Kolkata, West Bengal (IN)**
• **SHARMA, VARSHA**
**700160 Kolkata, West Bengal (IN)**
• **GHOSE, Avik**
**700156 Kolkata, West Bengal (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **SYSTEM AND METHOD OF PHOTOPLETHYSMOGRAPHY BASED HEART-RATE ESTIMATION IN PRESENCE OF MOTION ARTIFACTS**

(57) This disclosure relates to method for estimating heart rate associated with subject in presence of plurality of motion artifacts. The method includes receiving, a photoplethysmography signal and an acceleration signal associated with the subject; learning, by principal component analysis, a projection matrix by projecting input signal into n-dimensional subspaces to obtain a plurality of principal components; selecting, at least one principal component by (a) matching a dominant frequency of the principal components obtained from the PPG signal and a dominant frequency of the principal components obtained from the accelerometer signal, by applying a Fourier transform for a spectrum estimation; and (b) computing, at least one of (i) percentage of energy contributed by the principal component of the PPG signal, (ii) percentage of energy contributed by the principal component of the accelerometer signal; and estimating, the heart rate of the subject based on the at least one selected principal component.

FIG. 2

**Description**

CROSS-REFERENCE TO RELATED APPLICATIONS AND PRIORITY

The present application claims priority from Indian patent application no. 201921031018, filed on July 31, 2019.

TECHNICAL FIELD

The disclosure herein generally relates to physiological signal estimation, and, more particularly, to system and method of photoplethysmography based heart-rate estimation for a subject in presence of motion artifacts.

BACKGROUND

Generally, physiological estimation techniques could be characterized with three processing steps, namely, pre-processing, signal de-noising, and post-processing. Pre-processing step involves baseline removal and basic filtering; signal de-noising includes further noise cleaning; post-processing part integrates heart rate tracking and smoothing. So far, various methods has been investigated as effective de-noising methods, which are quite effective and caters better accuracy. However, since these algorithms are heavily dependent on the post-processing process and subsequently integrate several parameters tuned for the dataset, their generalization capability is limited. Moreover, computational load is another potential drawback while deploying the algorithm in a resource-constrained wearable hardware.

With emerging era of wearable technologies and smart watches, one active area of research that these devices have been used for, is longitudinal monitoring of physiological signals. The most pervasive physiological sensor available on wrist wearable devices is a Photoplethysmogram or PPG sensor. However, this optical way of measuring arterial pulse has a major drawback in being susceptible to motion artifacts due to ambulation. Any physiological sensing by the wearable biosensor gets distorted when it is subjected to the physical motion. The conventional filtering method is reliably effective when the frequency range of the motion is not overlapped with the true heart rate signal. Incidentally, the human movement is very low-frequency signal and coincides with actual heart rate signal (0.75 Hz to 3 Hz). Thus, when the strong motion signal overlaps with the signal of interest, estimating the heart rate in time or frequency domain becomes a challenging problem.

SUMMARY

Embodiments of the present disclosure present technological improvements as solutions to one or more of the above-mentioned technical problems recognized by the inventors in conventional systems. For example, in one aspect, processor implemented method for estimating heart rate associated with a subject in presence of plurality of motion artifacts is provided. The processor implemented method includes at least one of: receiving, from a first sensor, a first signal associated with the subject; receiving, from a second sensor, a second signal associated with the subject; filtering, a noise associated with at least one of the first sensor and the second sensor to discern cardiac signal by applying a same frequency range to the PPG signal and the acceleration signal; learning, by a principal component analysis (PCA), a projection matrix (W) by projecting input signal into n-dimensional subspaces to obtain a plurality of principal components; selecting, at least one principal component from the plurality of principal components, comprising: (a) matching a dominant frequency of the principal components obtained from the PPG signal and a dominant frequency of the principal components obtained from the accelerometer signal, by applying a Fourier transform for a spectrum estimation; and (b) computing, at least one of (i) a percentage of energy contributed by the principal component of the PPG signal, (ii) a percentage of energy contributed by the principal component of the accelerometer signal, or combination thereof; and estimating, the heart rate of the subject based on the at least one selected principal component. The first signal corresponds to a photoplethysmography (PPG) signal. The second signal corresponds to an acceleration signal of at least one axis along three axes. In an embodiment, corresponding resultant signal is a combined acceleration value of the at least one axis.

In an embodiment, the processor implemented method may further includes mapping original time series into a sequence of lagged vectors for a subspace decomposition. In an embodiment, at least one column of the projection matrix (W) may represent eigenvector computed from a covariance matrix ($C_H$). In an embodiment, eigenvectors of the covariance matrix ($C_H$) exploits a temporal covariance of the time series computed at different lags and represented as a Hankel matrix form. In an embodiment, at least one column of the projected matrix (Y) may corresponds to the plurality of principal components. In an embodiment, a resultant time series computed from the accelerometer signal is approximated by the plurality of principal components. In an embodiment, at least one of the plurality of principal components may be discarded if an absolute difference (d) is less than threshold. In an embodiment, the threshold may be defined as a frequency resolution provided by the Fourier Transform. In an embodiment, the percentage of energy may be estimated from eigenvalues obtained from an Eigen decomposition of the covariance matrix ($C_H$). In an embodiment, the processor implemented method may further includes classifying by a Decision Tree Classifier, to determine whether that the principal component is associated with a cardiac cycle.

In another aspect, there is provided a processor implemented system to estimate heart rate associated with a subject in presence of plurality of motion artifacts is provided. The system comprises a memory storing instructions; one or more communication interfaces; and one or more hardware processors coupled to the memory via the one or more communication interfaces, wherein the one or more hardware processors are configured by the instructions to: receive, from a first sensor, a first signal associated with the subject; receive, from a second sensor, a second signal associated with the subject; filter, a noise associated with at least one of the first sensor and the second sensor to discern cardiac signal by applying a same frequency range to the PPG signal and the acceleration signal; learn, by a principal component analysis (PCA), a projection matrix (W) by projecting input signal into n-dimensional subspaces to obtain a plurality of principal components; select, at least one principal component from the plurality of principal components comprising: (a) match a dominant frequency of the principal components obtained from the PPG signal and a dominant frequency of the principal components obtained from the accelerometer signal, by applying a Fourier transform for a spectrum estimation; and (b) compute, at least one of (i) a percentage of energy contributed by the principal component of the PPG signal, (ii) a percentage of energy contributed by the principal component of the accelerometer signal, or combination thereof; and estimating, the heart rate of the subject based on the at least one selected principal component. The first signal corresponds to a photoplethysmography (PPG) signal. The second signal corresponds to an acceleration signal of at least one axis along three axes. In an embodiment, corresponding resultant signal is a combined acceleration value of the at least one axis.

In an embodiment, the system may be further configured to map original time series into a sequence of lagged vectors for a subspace decomposition. In an embodiment, at least one column of the projection matrix (W) may represent eigenvector computed from a covariance matrix ($C_H$). In an embodiment, eigenvectors of the covariance matrix ($C_H$) exploits a temporal covariance of the time series computed at different lags and represented as a Hankel matrix form. In an embodiment, at least one column of the projected matrix (Y) may correspond to the plurality of principal components. In an embodiment, a resultant time series computed from the accelerometer signal is approximated by the plurality of principal components. In an embodiment, at least one of the plurality of principal components may be discarded if an absolute difference (d) is less than threshold. In an embodiment, the threshold may be defined as a frequency resolution provided by the Fourier Transform. In an embodiment, the percentage of energy may be estimated from eigenvalues obtained from an Eigen decomposition of the covariance matrix ($C_H$). In an embodiment, the system may be further configured to classify by a Decision Tree Classifier, to determine whether that the principal component is associated with a cardiac cycle.

In yet another aspect, there are provided one or more non-transitory machine readable information storage mediums comprising one or more instructions which when executed by one or more hardware processors causes at least one of: receiving, from a first sensor, a first signal associated with the subject; receiving, from a second sensor, a second signal associated with the subject; filtering, a noise associated with at least one of the first sensor and the second sensor to discern cardiac signal by applying a same frequency range to the PPG signal and the acceleration signal; learning, by a principal component analysis (PCA), a projection matrix (W) by projecting input signal into n-dimensional subspaces to obtain a plurality of principal components; selecting, at least one principal component from the plurality of principal components, comprising: (a) matching a dominant frequency of the principal components obtained from the PPG signal and a dominant frequency of the principal components obtained from the accelerometer signal by applying a Fourier transform for a spectrum estimation; and (b) computing, at least one of (i) a percentage of energy contributed by the principal component of the PPG signal, (ii) a percentage of energy contributed by the principal component of the accelerometer signal, or combination thereof; and estimating, the heart rate of the subject based on the at least one selected principal component. The first signal corresponds to a photoplethysmography (PPG) signal. The second signal corresponds to an acceleration signal of at least one axis along three axes. In an embodiment, corresponding resultant signal is a combined acceleration value of the at least one axis.

In an embodiment, the method may further include mapping original time series into a sequence of lagged vectors for a subspace decomposition. In an embodiment, at least one column of the projection matrix (W) may represent eigenvector computed from a covariance matrix ($C_H$). In an embodiment, eigenvectors of the covariance matrix ($C_H$) exploits a temporal covariance of the time series computed at different lags and represented as a Hankel matrix form. In an embodiment, at least one column of the projected matrix (Y) may correspond to the plurality of principal components. In an embodiment, a resultant time series computed from the accelerometer signal is approximated by the plurality of principal components. In an embodiment, at least one of the plurality of principal components may be discarded if an absolute difference (d) is less than threshold. In an embodiment, the threshold may be defined as a frequency resolution provided by the Fourier Transform. In an embodiment, the percentage of energy may be estimated from eigenvalues obtained from an Eigen decomposition of the covariance matrix ($C_H$). In an embodiment, the processor implemented method may further includes classifying by a Decision Tree Classifier, to determine whether that the principal component is associated with a cardiac cycle.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is a block diagram illustrating a system for photoplethysmography based heart-rate estimation in presence of a plurality of motion artifacts, according to embodiments of the present disclosure.
FIG. 2 is an exemplary physiological measurement system for heart rate estimation associated with a subject in presence of the plurality of motion artifacts, according to embodiments of the present disclosure.
FIG. 3 is an exemplary flow diagram illustrating a method for estimating heart rate associated with the subject in presence of plurality of motion artifacts according to embodiments of the present disclosure.

DETAILED DESCRIPTION OF EMBODIMENTS

Exemplary embodiments are described with reference to the accompanying drawings. In the figures, the leftmost digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following claims.

The embodiments of the present disclosure propose a photoplethysmography based estimation of heart rate associated with a subject in presence of motion artifacts. The embodiments of the present disclosure transform one or more raw signals into principal basis, instead of directly working on signal. Primarily, a PPG signal and simultaneously acquired accelerometer signal are discretized and approximated by a number of principal components. The principal components associated with motion or any other noise are discarded by one or more verification method i.e., (i) a dominant frequency of principal component is exploited as a similarity metric between the PPG signal and the accelerometer signal, and (ii) a machine learning based approach is considered for a principal component selection according to energy contribution features.

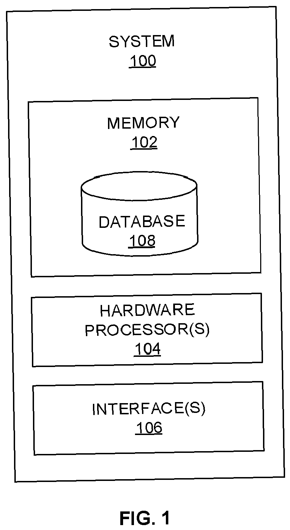

Referring now to the drawings, and more particularly to FIGS. 1 through 3, where similar reference characters denote corresponding features consistently throughout the figures, there are shown preferred embodiments and these embodiments are described in the context of the following exemplary system and/or method.

FIG. 1 is a block diagram illustrating a system 100 for photoplethysmography based heart-rate estimation in presence of a plurality of motion artifacts according to embodiments of the present disclosure. In an embodiment, the system 100 includes one or more processors 104, communication interface device(s) or input/output (I/O) interface(s) 106, and one or more data storage devices or memory 102 operatively coupled to the one or more processors 104. The memory 102 comprises a database 108. The one or more processors 104 that are hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes.

The database 108 may store information but are not limited to, a plurality of parameters obtained from one or more sensors, wherein the plurality of parameters are specific to an subject (e.g., a user, a patient, a machine, and the like). In an embodiment, the one or more sensors may be a temperature sensor, a motion sensor, a pressure sensor, a vibration sensor and the like. The parameters may include sensor data captured through the sensors either connected to the user and/or to the machine. Further, the database 108 stores information pertaining to inputs fed to the system 100 and/or outputs generated by the system 100 (e.g., data/output generated at each stage of the data processing), specific to the methodology described herein. More specifically, the database 108 stores information being processed at each step of the proposed methodology.

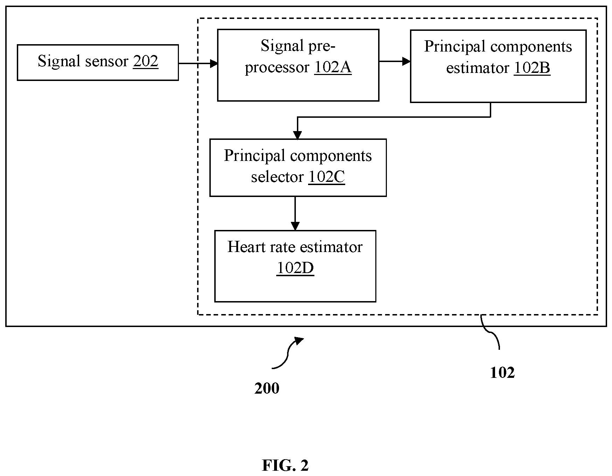

FIG. 2 illustrates an exemplary physiological measurement system 200 for heart rate estimation associated with a subject in presence of the plurality of motion artifacts according to embodiments of the present disclosure. The exemplary physiological measurement system 200 includes a signal sensor 202, and the memory 102. In an embodiment, the memory further includes a signal pre-processor 102A, a principal components estimator 102B, a principal components selector 102C, and a heart rate estimator 102D. The signal sensor 202 is configured to sense physical activity and physiological information of the subject being monitored. In an embodiment, the memory 102 interacts with the signal sensor 202 to receive one or more sensor inputs (e.g., the sensed physical activity and the physiological information). In another embodiment, the one or more processors 104 of the physiological measurement system 200 may include one or more modules configured with one or more instructions to estimate heart rate associated with a subject in presence of plurality of motion artifacts. The signal sensor 202 includes at least one of (i) a first sensor, and (ii) a second sensor. For example, the first sensor through which a first signal is obtained i.e., a photoplethysmography (PPG) signal. Similarly, the second sensor (e.g., a motion sensor) through which a second signal is obtained i.e., an acceleration signal of at least one axis along three axes (e.g., X, Y, Z axis respectively), and the resultant is at least a first direction.

In an embodiment, a physiological and a motion-related information includes sensed physical activity of a subject (e.g., user, patient) through the motion sensor attached to the subject, and sensing physiological information from the subject via the at least one photoplethysmography (PPG) sensor attached to the subject. In an embodiment, processing signals from the at least one motion sensor and signals from the at least one PPG sensor to generate a serial data string of physiological information and motion-related information. A plurality of subject physiological parameters can be extracted from the physiological information, and a plurality of subject physical activity parameters can be extracted from the motion-related information.

In an embodiment, the physical activity and the physiological information of the subject is sensed through a monitoring device (i.e., a wearable device) attached to the subject. For example, the monitoring device includes at least one motion sensor for sensing the physical activity and at least one photoplethysmography (PPG) sensor for sensing the physiological information. For example, the wearable device is configured to be attached to the subject at one or more of following body locations i.e., ear, limb, nose, and wrist. In an embodiment, the system 100 is configured to generate statistical relationships between subject physiological parameters and the physical activity parameters of the subject via at least one of the following techniques i.e., principal component analysis, multiple linear regression, and machine learning.

**Preprocessing:**

In an embodiment, preprocessing by the signal pre-processor 102A includes steps of at least one of a baseline removal, filtering process and the normalization. In an embodiment, frequency range of cardiac signal spans from 0.75Hz to maximum 3Hz, which encompasses heart beats per minute (BPM) from 42 BPM to 180 BPM. A band pass filter with same frequency range is applied to raw PPG signal to discern the cardiac signal. The filtering process subsequently eliminates sensor noise or any other noise outside of a signal of interest. Furthermore, the signal is normalized and a principal component analysis (PCA) and then a subspace learning based method are employed for subspace decomposition. A filtered signal, which is output of the filtering process, is subject to the normalization process. The normalization process is defined as:

$$Sig_{Norm} = \frac{FilteredSig - \mu}{\sigma} \qquad (1)$$

Where $\mu$ is mean of a signal window (e.g., accumulated signal for a specific time interval) and $\sigma$ is the standard deviation.

In an embodiment, the preprocessed signal is considered as an input for a subspace decomposition.

**Subspace Decomposition:**

(1) Hankel Matrix Conversion: The subspace decomposition is accomplished, when original time series is mapped into a sequence of lagged vectors. Consider a time series data X = {$x_1$, $x_2$, ..... $x_N$}, where N is the number of total samples; is transformed into L lagged vectors. The L is called as the window length and for a meaningful interpretation, L is chosen as $L < N/2$. A Trajectory matrix $TX \in R^{L*K}$ of the time series X is formed where $K = N - L + 1$.

$$X = TX_{i,j} = \begin{bmatrix} x_1 & x_2 & \cdots & x_L \\ x_2 & x_3 & \cdots & x_{L+1} \\ \vdots & \vdots & \ddots & \vdots \\ x_K & x_{K+1} & \cdots & x_N \end{bmatrix}$$

The trajectory matrix exhibits two important properties: (i) a diagonal of the matrix imparts the complete time series; moreover, rows and columns are the subseries of the actual time series; (ii) Cross-diagonals of TX is $x_{j+i=1}$ = $x_{i+j-1}$; and are considered as a Hankel Matrix.

**Principal Component Analysis (PCA):**

Considering a Hankel matrix $H \in R^{m*n}$, PCA aims to learn a projection matrix $W \in R^{n*n}$, projecting the input data into n-dimensional subspaces. Let Y is a projected matrix and denoted as:

$$Y = HW \qquad (2)$$

Where columns of the projection matrix W represent eigenvector computed from the covariance matrix of $HH^T$. The covariance matrix is defined as,

$$C_H = \frac{1}{N-1} HH^T \qquad (3)$$

Where N is a total number of samples.

In an embodiment, Eigenvectors are structured in ascending order and leading eigenvector is a last column of the projection matrix W. The eigenvectors (EOFs) of the covariance matrix $C_H$ exploits a temporal covariance of the time series, computed at different lags and represented as Hankel matrix form. The matrix Y is a projection of time series onto the Eigenvectors. The columns of the projected matrix Y are referred to as plurality of principal components. The plurality of principal components are again time series of same length of the Hankel matrix.

**Subspace Decomposition of Accelerometer Signal:**

In an embodiment, a motion subspace is approximated by the accelerometer signal. The acceleration sensor is configured to measure acceleration in three axes; $ACC \in R^3$, and resultant is computed and considered for further processing. After preprocessing, the subspace learning method is implied. Eventually, the resultant time series is approximated by number of principal components:

$$ACC_R \approx \sum_{K=1}^{N} PC_{ACC_R}(K) \qquad (4)$$

Where $ACC_R$ is the original time series accelerometer resultant signal and $PC_{ACC_R}(K)$ is PC vector.

**Selection of Principal Components:**

In an embodiment, the PPG signal is decomposed into orthogonal principal components, to discard the principal components which are contributed by the physical movement or other noise rather than one or more true cardiac cycles. To recognize the components associated with motion or noise, two-stage verification mechanism is employed. The two-stage verification mechanism includes a frequency-based similarity matching and a machine Learning Based Principal Component Selection.

(i) Frequency based similarity matching:

In an embodiment, uniformity between the principal component of PPG and accelerometer signal is established. Since the principal components are considered as the time series, spectrum estimation is exploited where the dominant frequency is utilized as a similarity metric. Formally, a Fourier Transform is applied on the spectrum estimation and are matched with the dominant frequency of the principal components obtained from PPG and accelerometer signal. The process is defined as

$$FQPPG_{max} = arg_k max\ FQSP_{PPG}\ (K) \qquad (5)$$

$$FQACC_{max} = arg_k max\ FQSP_{ACC}\ (K) \qquad (6)$$

$$d = |FQPPG_{max} - FQACC_{max}| \qquad (7)$$

Where $FQSP_{PPG}$ (K) and $FQSP_{ACC}$ (K) are the frequency spectrum of PPG and accelerometer signal respectively.

In an embodiment, if an absolute difference d is less than the threshold then the principal component is subjected for elimination. In an embodiment, the threshold is defined as a frequency resolution provided by the Fourier Transform. In order to remove unwanted noise, principal components where 90% of the energy is concentrated are considered.

$$X \approx \sum_{K=1}^{M} PC\ (K) \qquad (8)$$

Where X is the original PPG signal and M << N. The value of M is decided dynamically, according to the energy contribution of principal components.

A problem arises when PPG and motion signal coincide, and the dominant frequencies of both signals matched which leads to elimination of the principal component associated with true cardiac cycles. In order to avoid this kind of circumstances, along with similarity matching, energy contribution of the particular principal component is also considered. Further, second stage verification is applied where the machine learning based approach is considered for Principal Component selection.

(ii) Machine Learning Based Principal Component Selection: A principal component selection task are characterized as a classic type of two-category classification problem where the principal component is denoted as true or false. Based on an empirical analysis, is observed that energy contributed by the principal component of at least one signal is pivotal for ascertaining right principal components i.e.,

a) A percentage of energy contributed by the principal component of the PPG signal.
b) A percentage of energy contributed by the principal component of the accelerometer signal.

The energy is estimated from the eigenvalues obtained from the Eigen decomposition of covariance matrix and defined as:

$$Energy_{PC} = \frac{Eigen\ val_{PC}}{Sum\ (Eigen\ val diag)} * 100 \qquad (9)$$

Where $Eigen\ val_{PC} \in R^{n*1}$ is a diagonal vector of Eigen value matrix obtained from Eigen decomposition.

In an embodiment, a decision tree classifier is used as classification method to determine whether the principal component is associated with cardiac cycle or not.

After discarding, remaining principal components are considered for the signal reconstruction. In an embodiment, computing of the heart rate is performed, rather than transforming to the original time domain, clean signal is reconstructed by only adding the selected principal components.

$$CleanPPG = PC\ (i) + PC\ (j) + PC\ (k) + \ldots + PC\ (t) \qquad (10)$$

Where, i, j, k and t are arbitrary indexes chosen selectively.
In an embodiment, the clean PPG signal is considered as an input for heart rate estimation.

**Heart Rate Estimation Using Frequency Analysis:**

Inherently, rhythmic nature of the heart generates a pulsatile component in arterial blood which manifests a quasiperiodicity in the PPG signal. Essentially, estimation of heart rate is to find the periodicity of the PPG signal of a particular time window and subsequently compute for at least a minute. The frequency spectrum is obtained using the Fourier Transform, from which the dominant frequency with maximum amplitude is selected. Instead of using any extensive post-processing method, restricting search range of the frequency spectrum for the current window. The search range is computed using the previous estimation of the heart rate. The process is defined as follows:

$$FQPPG_{max} = \ argmax_{k \in [F_i,\dots,F_k]} PC_{PPG}\,(K) \qquad (11)$$

$$HR = FQPPG_{max} * 60 \qquad (12)$$

Where $F_i$ and $F_k$ are the range of the frequencies obtained according to the previous estimation.

**EXPERIMENTAL RESULTS:**

To demonstrate efficacy of the process of estimating the heart rate a specific set of data set is utilized. The dataset includes 12 training and 10 test data sets which were accumulated from 18 to 58 years old participants subjected to various physical activities. All sensor data are sampled at the 125HZ sampling rate. The physical activities include walking or running on a treadmill for various intervals and intensive forearm and upper arm exercise. For every participant i.e., the one or more subjects, two channels of PPG signals, three channels of simultaneous acceleration signals were acquired from a wrist worn device. Additionally, ECG signal is also obtained simultaneously from the chest using ECG sensors placed at the chest of the participant. A ground-truth heart rate is computed from the ECG signal which is utilized as the evaluation metric for the algorithm's performance.

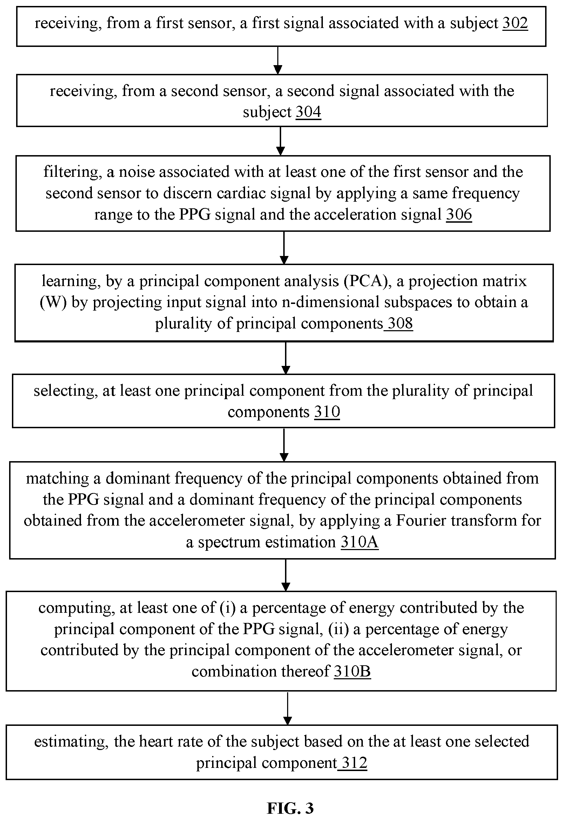

FIG. 3 is an exemplary flow diagram illustrating the method for estimating heart rate associated with the subject in presence of plurality of motion artifacts, using the system of FIG. 1, according to embodiments of the present disclosure. In an embodiment, the system 100 comprises one or more data storage devices or the memory 102 operatively coupled to the one or more hardware processors 104 and is configured to store instructions for execution of steps of the method by the one or more processors 104. The flow diagram depicted is better understood by way of following explanation/description.

The steps of the method of the present disclosure will now be explained with reference to the components of the system 100 as depicted in FIG. 1. At step 302, via the one or more hardware processors 104, a first signal associated with the subject is received from a first sensor. In an embodiment, the first signal is a photoplethysmography (PPG) signal. At step 304, via the one or more hardware processors 104, a second signal associated with the subject is received from a second sensor. The second signal corresponds to an acceleration signal of at least one axis along three axes (e.g., X, Y, Z axis respectively). At step 306, via the one or more hardware processors 104, a noise associated with the first sensor and the second sensor is filtered to discern cardiac signal by applying a same frequency range to the PPG signal and the acceleration signal.

At step 308, via the one or more hardware processors 104, a projection matrix (W) is learned by a principal component analysis (PCA), i.e. by projecting input signal into n-dimensional subspaces to obtain a plurality of principal components. At step 310, via the one or more hardware processors 104, at least one principal component is selected from the plurality of principal components by at least one step i.e. (i) at step 310a, a Fourier transform for a spectrum estimation is applied, to match a dominant frequency of the principal components obtained from the PPG signal and a dominant frequency of the principal components obtained from the accelerometer signal, and (ii) at step 310b, at least one of (a) a percentage of energy contributed by the principal component of the PPG signal, or (b) a percentage of energy contributed by the principal component of the accelerometer signal, or a combination thereof is computed. At step 312, via the one or more hardware processors 104, the heart rate of the subject is estimated based on the at least one selected principal component.

The method further includes mapping of original time series into a sequence of lagged vectors for a subspace decomposition. In an embodiment, at least one column of the projection matrix (W) represents eigenvector computed from a covariance matrix ($C_H$). In an embodiment, eigenvectors of the covariance matrix ($C_H$) exploits a temporal covariance of the time series computed at different lags and represented as a Hankel matrix form. In an embodiment, at least one column of the projected matrix (Y) corresponds to the plurality of principal components.

In an embodiment, a resultant time series computed from the accelerometer signal is approximated by the plurality of principal components. In an embodiment, the principal component associated with a cardiac cycle is determined based on a Decision Tree Classifier. In an embodiment, at least one principal component is discarded if an absolute difference (d) is less than threshold, wherein the threshold is defined as a frequency resolution provided by the Fourier Transform. In an embodiment, the energy is estimated from an eigenvalues obtained from an Eigen decomposition of the covariance matrix ($C_H$). In an embodiment, the corresponding resultant signal is a combined acceleration value of the at least one axis.

The embodiments of present disclosure provide a subspace based, low-complexity algorithm that can be used in real-time and on-premise to achieve an accuracy comparable to more complex approaches. The motion artifact is additive in nature and the acquired signal is an aggregate of both true heart signal and the motion signal. Since significant portion of motion artifacts is not directly correlated to the actual PPG signal, this problem is aligned to subspace learning method where two major components, motion signal and the true heart rate signal lies in two distinct subspaces. The main objective is to distinguish the motion signal subspace and eventually recover signal of interest. Principal Component Analysis (PCA) is employed as a subspace learning method which transforms original time series signal into principal subspaces. Generally, PCA is utilized as a dimensionality reduction technique. However, it could be used as a de-noising method by suitable selection of principal components. Essentially, the PCA approximates the original time series signal into a number of constitutive principal components.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method for estimating heart rate associated with a subject in presence of plurality of motion artifacts, comprising:

> receiving, from a first sensor, a first signal associated with the subject, wherein the first signal corresponds to a photoplethysmography (PPG) signal;
> receiving, from a second sensor, a second signal associated with the subject, wherein the second signal corresponds to an acceleration signal of at least one axis along three axes, wherein corresponding resultant signal is a combined acceleration value of the at least one axis;
> filtering, a noise associated with at least one of the first sensor and the second sensor to discern cardiac signal by applying a same frequency range to the PPG signal and the acceleration signal;
> learning, by a principal component analysis (PCA), a projection matrix (W) by projecting input signal into n-dimensional subspaces to obtain a plurality of principal components;
> selecting, at least one principal component from the plurality of principal components, comprising:
>
>> (a) matching a dominant frequency of the principal components obtained from the PPG signal and a dominant frequency of the principal components obtained from the accelerometer signal, by applying a Fourier transform for a spectrum estimation; and
>> (b) computing, at least one of (i) a percentage of energy contributed by the principal component of the PPG signal, (ii) a percentage of energy contributed by the principal component of the accelerometer signal, or combination thereof; and
>
> estimating, the heart rate of the subject based on the at least one selected principal component.

2. The method of claim 1, further comprising, mapping original time series into a sequence of lagged vectors for a subspace decomposition.

3. The method of claim 1, wherein at least one column of the projection matrix (W) represents eigenvector computed from a covariance matrix ($C_H$), wherein eigenvectors of the covariance matrix ($C_H$) exploits a temporal covariance of the time series computed at different lags and represented as a Hankel matrix form, wherein at least one column of the projected matrix (Y) corresponds to the plurality of principal components, wherein a resultant time series computed from the accelerometer signal is approximated by the plurality of principal components.

4. The method of claim 1, wherein at least one of the plurality of principal components is discarded if an absolute difference (d) is less than threshold, wherein the threshold is defined as a frequency resolution provided by the Fourier Transform.

5. The method of claim 1, wherein the percentage of energy is estimated from eigenvalues obtained from an Eigen decomposition of the covariance matrix ($C_H$).

6. The method of claim 1, further comprising, classifying by a Decision Tree Classifier, to determine whether that the principal component is associated with a cardiac cycle.

7. A system (100) to estimate heart rate associated with a subject in presence of plurality of motion artifacts, wherein the system comprising:

> a memory (102) storing instructions;
> one or more communication interfaces (106); and
> one or more hardware processors (104) coupled to the memory (102) via the one or more communication interfaces (106), wherein the one or more hardware processors (104) are configured by the instructions to:
>
>> receive, from a first sensor, a first signal associated with the subject, wherein the first signal corresponds to a photoplethysmography (PPG) signal;

receive, from a second sensor, a second signal associated with the subject, wherein the second signal corresponds to an acceleration signal of at least one axis along three axes, wherein corresponding resultant signal is a combined acceleration value of the at least one axis;

filter, a noise associated with at least one of the first sensor and the second sensor to discern cardiac signal by applying a same frequency range to the PPG signal and the acceleration signal;

learn, by a principal component analysis (PCA), a projection matrix (W) by projecting input signal into n-dimensional subspaces to obtain a plurality of principal components;

select, at least one principal component from the plurality of principal components, comprising:

(a) match, a dominant frequency of the principal components obtained from the PPG signal and a dominant frequency of the principal components obtained from the accelerometer signal, by applying a Fourier transform for a spectrum estimation; and

(b) compute, at least one of (i) a percentage of energy contributed by the principal component of the PPG signal, (ii) a percentage of energy contributed by the principal component of the accelerometer signal, or combination thereof; and

estimate, the heart rate of the subject based on the at least one selected principal component.

**8.** The system (100) of claim 7, wherein the one or more hardware processors are further configured to map original time series into a sequence of lagged vectors for a subspace decomposition.

**9.** The system (100) of claim 7, wherein at least one column of the projection matrix (W) represents eigenvector computed from a covariance matrix ($C_H$), wherein eigenvectors of the covariance matrix ($C_H$) exploits a temporal covariance of the time series computed at different lags and represented as a Hankel matrix form, wherein at least one column of the projected matrix (Y) corresponds to the plurality of principal components, wherein a resultant time series computed from the accelerometer signal is approximated by the plurality of principal components.

**10.** The system (100) of claim 7, wherein at least one of the plurality of principal components is discarded if an absolute difference (d) is less than threshold, wherein the threshold is defined as a frequency resolution provided by the Fourier Transform.

**11.** The system (100) of claim 7, wherein the percentage of energy is estimated from eigenvalues obtained from an Eigen decomposition of the covariance matrix ($C_H$).

**12.** The system (100) of claim 7, wherein the one or more hardware processors are further configured to classify, by a Decision Tree Classifier, to determine whether that the principal component is associated with a cardiac cycle.

**13.** One or more non-transitory machine-readable information storage mediums comprising one or more instructions which when executed by one or more hardware processors cause:

receiving, from a first sensor, a first signal associated with the subject, wherein the first signal corresponds to a photoplethysmography (PPG) signal;

receiving, from a second sensor, a second signal associated with the subject, wherein the second signal corresponds to an acceleration signal of at least one axis along three axes, wherein corresponding resultant signal is a combined acceleration value of the at least one axis;

filtering, a noise associated with at least one of the first sensor and the second sensor to discern cardiac signal by applying a same frequency range to the PPG signal and the acceleration signal;

learning, by a principal component analysis (PCA), a projection matrix (W) by projecting input signal into n-dimensional subspaces to obtain a plurality of principal components;

selecting, at least one principal component from the plurality of principal components, comprising:

(a) matching a dominant frequency of the principal components obtained from the PPG signal and a dominant frequency of the principal components obtained from the accelerometer signal, by applying a Fourier transform for a spectrum estimation; and

(b) computing, at least one of (i) a percentage of energy contributed by the principal component of the PPG signal, (ii) a percentage of energy contributed by the principal component of the accelerometer signal, or combination thereof; and

estimating, the heart rate of the subject based on the at least one selected principal component.

14. The one or more non-transitory machine-readable information storage mediums of claim 13, wherein the step of estimating heart rate associated with a subject in presence of plurality of motion artifacts comprises:

   mapping, original time series into a sequence of lagged vectors for a subspace decomposition; and
   classifying, by a decision tree classifier, to determine whether that the principal component is associated with a cardiac cycle.

15. The one or more non-transitory machine-readable information storage mediums of claim 13, wherein at least one column of the projection matrix (W) represents eigenvector computed from a covariance matrix ($C_H$), wherein eigenvectors of the covariance matrix ($C_H$) exploits a temporal covariance of the time series computed at different lags and represented as a Hankel matrix form, wherein at least one column of the projected matrix (Y) corresponds to the plurality of principal components, wherein a resultant time series computed from the accelerometer signal is approximated by the plurality of principal components, wherein at least one of the plurality of principal components is discarded if an absolute difference (d) is less than threshold, wherein the threshold is defined as a frequency resolution provided by the Fourier Transform, wherein the percentage of energy is estimated from eigenvalues obtained from an Eigen decomposition of the covariance matrix ($C_H$).

SYSTEM
100

MEMORY
102

DATABASE
108

HARDWARE
PROCESSOR(S)
104

INTERFACE(S)
106

FIG. 1

FIG. 2

receiving, from a first sensor, a first signal associated with a subject 302

receiving, from a second sensor, a second signal associated with the subject 304

filtering, a noise associated with at least one of the first sensor and the second sensor to discern cardiac signal by applying a same frequency range to the PPG signal and the acceleration signal 306

learning, by a principal component analysis (PCA), a projection matrix (W) by projecting input signal into n-dimensional subspaces to obtain a plurality of principal components 308

selecting, at least one principal component from the plurality of principal components 310

matching a dominant frequency of the principal components obtained from the PPG signal and a dominant frequency of the principal components obtained from the accelerometer signal, by applying a Fourier transform for a spectrum estimation 310A

computing, at least one of (i) a percentage of energy contributed by the principal component of the PPG signal, (ii) a percentage of energy contributed by the principal component of the accelerometer signal, or combination thereof 310B

estimating, the heart rate of the subject based on the at least one selected principal component 312

**FIG. 3**

Europäisches
Patentamt

European
Patent Office

Office européen
des brevets

# EUROPEAN SEARCH REPORT

Application Number

EP 20 18 8493

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AHMED NASIMUDDIN ET AL: "Heart Rate Estimation Algorithm From Wrist-based Photoplethysmogram Using Subspace Learning Method", 2019 IEEE INTERNATIONAL CONFERENCE ON PERVASIVE COMPUTING AND COMMUNICATIONS WORKSHOPS (PERCOM WORKSHOPS), IEEE, 11 March 2019 (2019-03-11), pages 145-150, XP033558310, DOI: 10.1109/PERCOMW.2019.8730797 [retrieved on 2019-06-04] * the whole document * | 1-15 | INV. A61B5/024 A61B5/00 |
| A | US 2016/051157 A1 (WAYDO STEPHEN J [US]) 25 February 2016 (2016-02-25) * abstract * * paragraph [0003] * * figures 1,2 * | 1,7,13 | |
| A | GALLI A ET AL: "Robust estimation and tracking of heart rate by PPG signal analysis", 2017 IEEE INTERNATIONAL INSTRUMENTATION AND MEASUREMENT TECHNOLOGY CONFERENCE (I2MTC), IEEE, 22 May 2017 (2017-05-22), pages 1-6, XP033114946, DOI: 10.1109/I2MTC.2017.7969715 [retrieved on 2017-07-05] * the whole document * | 1,7,13 | TECHNICAL FIELDS SEARCHED (IPC) A61B |

The present search report has been drawn up for all claims

| Place of search | Date of completion of the search | Examiner |
|---|---|---|
| The Hague | 24 November 2020 | Van der Haegen, D |

EPO FORM 1503 03.82 (P04C01)

## ANNEX TO THE EUROPEAN SEARCH REPORT
## ON EUROPEAN PATENT APPLICATION NO.

EP 20 18 8493

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2020

| Patent document cited in search report | Publication date | Patent family member(s) | Publication date |
|---|---|---|---|
| US 2016051157 A1 | 25-02-2016 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 201921031018